# EUROPÄISCHE PATENTSCHRIFT

(11) **EP 0 456 106 B1**
(45) Veröffentlichungstag und Bekanntmachung des Hinweises auf die Patenterteilung: **07.12.1994**
(21) Anmeldenummer: 91107066.2
(22) Anmeldetag: 02.05.1991
(51) Int. Cl.: A61K 9/107, A61K 31/275

(54) **Stabile, zur pharmazeutischen Verabreichung geeignete Emulsion, Verfahren zu deren Herstellung und Emulsion zur Anwendung als Arzneimittel**
Stable emulsion for pharmaceutical administration, process for its preparation and use thereof as pharmaceutical
Emulsion stable pour l'administration pharmaceutique, procédé pour sa préparation et son emploi comme agent pharmaceutique

(30) Priorität: 11.05.1990 DE 4015108
(43) Veröffentlichungstag der Anmeldung: 13.11.1991
(73) Patentinhaber: KNOLL AG, D-67061 Ludwigshafen (DE)
(72) Erfinder: Schaupp, Karin, Dr., A-8010 Graz (AT); Polzer, Josef, A-8053 Graz (AT); Kerbl, Johannes, Dr., A-8052 Graz (AT); Lanthaler, Kurt, A-8761 Poels (AT); Davis, Stanley Stewart, Prof., The Park, Nottingham (GB); Washington, Clive, Dr., Toton, Nottingham (GB)
(74) Vertreter: Karau, Wolfgang, Dr.

(56) Entgegenhaltungen:
- EP-A- 0 211 258
- EP-A- 0 276 735
- WO-A-91/02517
- US-A- 4 592 859
- CHEMICAL ABSTRACTS, vol. 89, no. 14, 2. Oktober 1978, Columbus, Ohio, US;abstract no. 117634P, W. H. DAWES ET AL: 'The effect of electrolytes onphospholipid-stabilised soybean oil emulsions'
- KIRK-OTHMER ENCYCLOPEDIA OF CHEMICAL TECHNOLOGY 3rd Ed., 1984, SupplementVolume WILEY-INTERSCIENCE, NY A: BLEIER "Colloids" , p.241-259

## Beschreibung

Die vorliegende Erfindung betrifft eine Emulsion vom Typ Öl-in-Wasser, die einen hydrophoben, basischen Wirkstoff in der Ölphase gelöst enthält. Sie ist zur pharmazeutischen, insbesondere intravenösen Verabreichung geeignet und zeichnet sich durch sehr gute Stabilität aus, die auf einer elektrostatischen Abstoßung der dispersen Phase beruht.

Aus der DE-B 17 92 410 ist bekannt, daß Arzneimittelzubereitungen, in denen der zu verabreichende Wirkstoff in der hydrophoben Phase eines Systems vom Typ Lipid-in-Wasser fein verteilt oder insbesondere gelöst vorliegt, für die intravenöse Verabreichung sehr gut geeignet sind und sich häufig gegenüber wäßrigen Darreichungsformen des gleichen Wirkstoffes durch bessere Verträglichkeit und geringere Nebenwirkungen auszeichnen. In vielen Fällen wurde bei diesen Zubereitungen auch eine Erhöhung der Wirksamkeit beobachtet. Zur Stabilisierung des als Träger dienenden Lipid-in-Wasser-Systems dienen hierbei stabilisierende Stoffe natürlichen oder synthetischen Ursprungs wie Phosphatide, Polypropylen-Polyäthylenglycol, Polyglycerin-Monooleat, deren eingesetzte Menge laut Beschreibung von den jeweiligen Eigenschaften des Systems abhängen. In den Beispielen wird ein Gemisch von Phosphatiden, wie Eiphosphatid, mit nichtionischen Emulgatoren, wie z.B. Polyoxyäthylen, das zum Teil mit Stearinsäure verestert ist, verwendet, wobei in 10 % Öl-in-Wasser-Emulsionen 1-2 Gew.-% Phosphatid zusammen mit etwa 0,5 Gew.-% des nichtionischen Emulgators zum Einsatz kommen.

Im Gegensatz zu den Emulsionen gemäß DE-B 17 92 410, bei denen keine organischen Lösungsmittel zur Anwendung kommen, gibt es auch Vorschläge zur Herstellung von Darreichungsformen für hydrophobe Wirkstoffe in Form von Fettemulsionen, bei denen organische Lösungsmittel als Hilfsstoffe eingesetzt werden. So wird gemäß US-A 4,784,845 vorgeschlagen, zur Herstellung von Fettemulsionen von u.a. solchen basischen Wirkstoffen, deren pKa-Wert unterhalb oder nahe dem physiologischen pH-Wert liegt, die also nur sehr schwach basisch sind, Benzylalkohol als "Cosolvent" einzusetzen und wird gemäß DE-A 37 02 029 für die Herstellung von Fettemulsionen mit u.a. 2-Dodecyl-5-(methyl-3'-methoxyphenethylamino)-2-3'-methoxy-phenylvaleronitril (Anipamil) als Wirkstoff mittels eines komplizierten Verfahrens die Mitverwendung von Isopropanol empfohlen, wobei dieses Lösungsmittel erst nach Zuführung eines Schutzkolloids wie z.B. Gelatine zugleich mit der Hauptmenge des Wassers aus dem System entfernt wird.

Das daraus resultierende Konzentrat enthält dann nur mehr geringe Mengen Wasser und ist nicht mehr als Lipid-in-Wasser-Emulsion im Sinne der vorliegenden Erfindung anzusprechen.

Solchen Emulsionen mit einem Gehalt an organischen Lösungsmitteln sind Emulsionen, die nur aus einer Öl-in-Wasser-Emulsion mit lösungsmittelfreier, wäßriger Phase und Phospholipiden als Emulgator bestehen, vorzuziehen, vor allem dann, wenn sie intravenös verabreicht werden, da letztere den Chylomicronen ähnlich sind, die dem physiologischen Fetttransport im Blut dienen und in der Membran Phospholipide enthalten (siehe die DE-B 17 92 410). Sie sind daher vom physiologischen Standpunkt unbedenklicher.

Auch Davis, S. S., et al., Ann. New York Acad. Sci 507, Seite 76-79 (1987), die dort auf die Bedeutung, die Emulsionen vom Typ Öl-in-Wasser als Verabreichungsform für öllösliche, nicht aber wasserlösliche pharmazeutische Wirkstoffe erlangt haben, hinweisen, heben ebenfalls hervor, daß solche Emulsionen, die meist aus 10-20 Vol-% eines pflanzlichen Öls, das durch 1-2 Gew.-% an Phosphatiden stabilisiert ist, bestehen, den Chylomicronen ähnlich und Systemen auf Basis organischer Lösungsmittel und synthetischer, oberflächenaktiver Mittel, wie z.B. Cremophor, überlegen sind, da sie besser intravenös verträglich sind.

Diese Emulsionen besitzen auch eine gewisse Ähnlichkeit mit Liposomen, zeichnen sich gemäß Davis S. S., et al. diesen gegenüber aber durch eine einfachere, wohlerprobte Herstellbarkeit und eine gute Lagerstabilität aus. Vorteilhaft ist, daß solche Fettemulsionen auch als Darreichungsform für solche Wirkstoffe dienen können, die in wäßrigem Medium instabil sind oder in wäßrigen Darreichungsformen unerwünschte Nebenwirkungen zeigen. Als Nachteil wird hingegen gewertet, daß manche Wirkstoffe die Stabilität der Basisemulsion entscheidend beeinträchtigen bzw. die Emulsion überhaupt zerstören. Eine Ursache für die negative Wirkung mancher Wirkstoffe wird nicht angegeben.

In Adv. Clin. Nutrition - Proc. 2^{nd} Int. Symp. Editio I.D.A. Johnston 1982, Seiten 213-239 hat S. S. Davis ferner darauf hingewiesen, daß die stabilisierende Wirkung von Emulgatoren sowohl mechanischer als auch elektrischer Natur sein kann, wobei die Stabilisierung auf mechanischem Wege auf der Ausbildung eines dicken Filmes an der Grenzfläche, die Stabilisierung auf elektrischem Wege auf einer elektrostatischen Abstoßung der Tröpfchen der dispersen Phase durch gleichsinnige Aufladung an der Grenzfläche beruht. Für die parenterale Verabreichung werden in erster Linie Fettemulsionen mit Lecithin tierischen oder pflanzlichen Ursprungs als Emulgator eingesetzt, die aufgrund geringer saurer Beimengungen wie Phosphatidsäure, Phosphatidylserin und ähnlichen Substanzen den Fetttröpfchen eine negative Ladung verleihen, die für die Stabilität solcher Emulsionen ausschlaggebend ist. Diese elektrostatischen Kräfte können durch das Zetapotential der Fetttröpfchen ausgedrückt werden, das ist gemäß Römpps Chemie Lexikon, Otto Albrecht Neumüller - Stuttgart: Franckh, Bd. 6, 8. Auflage, Seite 4695, das nach außen wirksame Potential der Teilchen, das für deren elektrokinetische Erscheinungen verantwortlich ist und deshalb auch elektrokinetisches Potential genannt wird. Je größer das negative Zetapotential ist, desto stabiler ist die Emulsion.

Davis weist ferner darauf hin, daß der Zusatz von Kationen das negative Zetapotential der Fettemulsionen, das bei handelsüblichen Fettemulsionen bei -40 bis -50 mV liegt, herabsetzt und so zur Instabilität der Emulsion führt. Bei einwertigen Kationen, wie z.B. Natrium- oder Kaliumion, bewirkt ein Zusatz von mehr als 130 mmol/l eine Zerstörung der Emulsion, bei zwei- oder gar dreiwertigen Kationen tritt der zerstörende Effekt bereits bei wesentlich niedrigeren Konzentrationen auf. Bei Zusatz der mehrwertigen Kationen kann es sogar bis zu einer völligen Entladung der Fetttröpfchen bzw. zu einer Umladung zu einem positiven Zetapotential kommen.

Die vorliegende Erfindung beruht auf der Feststellung, daß es in der Regel basische Wirkstoffe sind, die die Instabilität von Fettemulsionen, die diesen Wirkstoffen als Träger dienen sollten, bewirken. Dieser instabilisierende Effekt nimmt mit steigendem pKa-Wert des basischen Wirkstoffes zu. So können zwar mit schwach basischen Wirkstoffen, wie etwa Diazepam oder schwach sauren Wirkstoffen, wie Propofol, stabile Arzneimittelzubereitungen auf Basis von Fettemulsionen hergestellt werden, und es zeigte sich, daß dies damit zusammenhängt, daß durch den Zusatz dieser genannten Wirkstoffe das negative Zetapotential der als Basis dienenden Fettemulsion nur wenig oder gar nicht verändert wird. Bei stärker basisch reagierenden Wirkstoffen, inbsbesondere bei solchen mit einem pKa-Wert von 8 oder höher konnte hingegen überraschenderweise festgestellt werden, daß bei pH 7 die positive Ionisation des Wirkstoffes trotz seiner Unlöslichkeit in Wasser in einer 10 %igen Sojaöl-Emulsion bereits ausreichen kann, um die negative Ladung an der Grenzfläche der Fetttröpfchen nahezu zu neutralisieren oder sogar eine gewisse Umladung zu erzielen und damit die stabilisierend wirkende elektrostatische Abstoßung auszuschalten. So zeigte sich z.B. im Falle des bekannten Calcium-Antagonisten (-)-(S)-2-Isopropyl-5-(methylphenethylamino)-2-phenyl-valeronitril (Levemopamil), der extrem hydrophob [Verteilungskoeffizient (Octanol/Wasser, nicht ionisiert) Log P 6-7, bestimmt durch Kalkulation] ist und einen pKa-Wert von 8,58 besitzt, daß der ionisierte Anteil von 2 % in einer 10 % Sojaöl enthaltenden Emulsion bereits bewirkt, daß das Zetapotential der Emulsion, das ohne Wirkstoffgehalt -40 mV ergibt, bei pH 7 auf -10 bis +5 mV geändert wird, was Anlaß für die Instabilität der Arzneimittelzubereitung mit diesem Wirkstoff ist.

Überraschenderweise konnte nun gefunden werden, daß sich hydrophobe, stark basische Wirkstoffe dann zu stabilen Arzneimittelzubereitungen auf Basis einer mit Phospholipid stabilisierten Öl-in-Wasser-Emulsion formulieren lassen, wenn in der wäßrigen Phase durch Zugabe eines Puffersystems ein pH-Wert im sauren Bereich eingestellt wird, der ausreicht, um eine Umladung zu hohen positiven Zetapotentialen zu bewirken. Durch die Einstellung eines sauren pH-Wertes in der wäßrigen Phase, beispielsweise auf pH 6 oder darunter, wird die Ionisation des in Wasser schwer löslichen Wirkstoffes so stark erhöht, daß das Zetapotential der dispersen Phase bei Aufrechterhaltung der Öllöslichkeit so weit in den positiven Bereich verschoben wird, daß die abstoßenden Kräfte der nunmehr positiv geladenen Fetttröpfchen ausreichen, um die Emulsion zu stabilisieren. Dies ist überraschend, da dieser Effekt bereits bei relativ niedrigen Wirkstoffkonzentrationen, bezogen auf das Gesamtsystem, eintritt. So genügen z.B. bei Levemopamil 2 % Wirkstoff, bezogen auf das Gesamtsystem, das sind 60 mmol/l, von denen nur ein Bruchteil ionisiert ist, um bei pH 6 ein positives Zetapotential von etwa +35 bis +40 mV zu ergeben. Durch Einstellung eines pH-Wertes von 5 läßt sich das Zetapotential sogar auf über +50 mV steigern.

Ausschlaggebend für das Eintreten des stabilisierenden Effektes sind naturgemäß die elektrostatischen Verhältnisse in der verabreichungsfertigen Emulsion. In der Praxis hat es sich daber gezeigt, daß es nicht möglich ist, an diesen Emulsionen, die 5-30 Gew.-% Fett enthalten, Messungen des Zetapotentials, beispielsweise durch Messung der Wanderungsgeschwindigkeit durch Microelektrophorese, gekoppelt mit einer Laser-Doppler-Velocimetrie, durchzuführen, da die Transparenz dieser Emulsionen zu gering ist. Andererseits besteht im vorliegenden Fall die Schwierigkeit, daß das in der fertigen Zubereitung sich ergebende positive Zetapotential nicht auf das Vorhandensein von Fremdelektrolyten in der wäßrigen Phase zurückzuführen ist, sondern sich diese Elektrolyte durch Ionisation des in der Ölphase befindlichen Wirkstoffes bilden, die durch eine Verdünnung der Probe erhöht wird. Der Einfluß der zur Messung nötigen Verdünnung ist dabei so gravierend, daß, wie eigene Untersuchungen gezeigt haben, Emulsionen, deren Zetapotential etwa zwischen +40 und +60 mV liegt, bei Verdünnungen von 1 : 4.000 bis 1 : 10.000 den Nullpunkt erreichen oder gar in negative Werte abrutschen. Es zeigte sich jedoch, daß sich Zetapotentiale mit im Handel befindlichen Laser Electrophoresis and Submicron Particle Size Distribution Analysern bei Fettkonzentrationen von 0,08 Gew.-% sehr gut messen lassen und die so erhaltenen Werte, wie aufgrund des Verlaufes der Verdünnungskurve ersichtlich, sehr gut mit jenen der unverdünnten Emulsion korrelieren. Siehe dazu Tab. 1, Verlauf der Zetapotentiale von Verdünnungen mit 0,08 Gew.-% bis 0,0002 Gew.-% Öl. Erhält man bei Messung einer 0,08 gew.-%igen Emulsion ein positives Zetapotential von mindestens +15 mV, so hat man, wie die Praxis ergab, den stabilen Bereich für diese Emulsion erreicht, wobei für die Sicherstellung einer Langzeitstabilität und Autoklavierbarkeit bei 120°C zu empfehlen ist, Emulsionen zu bereiten, bei denen sich bei Messung an der auf einen Fettgehalt von 0,08 Gew.-% verdünnten, verabreichungsfertigen Zubereitung ein Zetapotential von mindestens +30 mV, bevorzugt von mindestens +40 mV, ergibt.

Diese 0,08 Gew.-% Fett enthaltenden Emulsionen werden z.B. erhalten, wenn eine ursprünglich
20 gew.-%ige Fettemulsion auf das 250fache, eine
10 gew.-%ige Fettemulsion auf das 125fache und eine
5 gew.-%ige Fettemulsion auf das 62,5fache Volumen verdünnt wird.

Alle in der folgenden Beschreibung angegebenen positiven Zetapotentiale sind daher an 0,08 Gew.-% Fett enthaltenden Emulsionen bestimmt worden.

Gegenstand der vorliegenden Erfindung sind demnach stabile, zur pharmazeutischen, insbesondere intravenösen Verabreichung geeignete Emulsionen vom Typ Öl-in-Wasser mit einem Fettgehalt von 5-30 Gew.-% und einem Gehalt an 0,5-2 Gew.-% eines Phospholipids als Emulgator, die in der Lipidphase einen eine oder mehrere basische Gruppen tragenden, hydrophoben, pharmazeutischen Wirkstoff in fein verteilter und/oder gelöster Form enthalten, die dadurch gekennzeichnet sind, daß der Wirkstoff öllöslich ist und einen pKa-Wert von mindestens 7,5 aufweist, die wäßrige Phase durch Gehalt eines physiologisch verträglichen Puffersystems auf einen pH-Wert im sauren Bereich eingestellt ist und die disperse Phase nach Verdünnung der verabreichungsfertigen Emulsion auf einen Fettgehalt von 0,08 Gew.-% ein positives Zetapotential von mindestens +15 mV ergibt.

Die Höhe des positiven Zetapotentials der verabreichungsfertigen Emulsion ist für die Stabilität der Emulsion verantwortlich, da ja erst nach Erreichen einer positiven Mindestaufladung an der Grenzfläche die abstoßenden Kräfte so groß werden, daß ein Aufrahmen oder eine Ölbildung verhindert werden. Welcher pH-Wert in der wäßrigen Phase genau eingestellt werden muß, um die geforderte Grenze von +15 mV zu überschreiten, hängt dabei nicht nur von der Basizität des Wirkstoffes, ausgedrückt durch dessen pKa-Wert, ab, sondern auch in gewissem Ausmaß von der Konzentration des Wirkstoffes in der Zubereitung, das heißt, je höher die Konzentration, desto geringer ist die Acidität, die erforderlich ist, um die angestrebte positive Ladung der dispersen Phase zu erreichen. Schließlich geht auch noch das verwendete Lecithin ein, die negativen Zetapotentiale von Lecithinen können ja je nach Reinheitsgrad, gemessen an einer wirkstofffreien Fettemulsion bei pH 7,4, zwischen -40 bis -50 mV und nahe 0 (bei reinem Phosphatidylcholin) schwanken.

Letztlich ist die Ionenkonzentration in der wäßrigen Phase unter Aufrechterhaltung der Öllöslichkeit des Wirkstoffes maßgebend, die sich durch Zusammenwirken aller dieser Faktoren ergibt. Dabei ist davon auszugehen, daß der Wert von +15 mV die untere Grenze für eine Stabilität der Zubereitung darstellt.

Bevorzugt ist ein positives Zetapotential von mindestens +30 mV und besonders bevorzugt ein solches von mindestens +40 mV anzustreben, vor allem dann, wenn eine Autoklavierung der Zubereitung vorgesehen ist.

Wichtig ist neben der Öllöslichkeit auch eine möglichst ausgeprägte Hydrophobie des Wirkstoffes, wobei auch hier zu gelten hat, daß eine hohe Hydrophobie die Ausbildung eines hohen, positiven Zetapotentials begünstigt, gleichzeitig aber gewährleistet, daß die Hauptmenge des Wirkstoffes in der Ölphase verbleibt. Es ist davon auszugehen, daß bei einem Verteilungskoeffizienten Log P, gemessen im System Octanol/Wasser, nicht ionisiert, von wesentlich höher als 2,5 bis 3 von starker Hydrophobie gesprochen werden kann, wobei sich die Grenze von 2,5 bis 3 durch die Nachweisgrenze vieler Wirkstoffe ergibt. Durch Kalkulation ermittelte Werte von Log P sollten dabei bevorzugt über 4 betragen.

Es empfiehlt sich, für einen gewählten Wirkstoff durch einen Vorversuch den pH-Wert zu ermitteln, bei dem sich ein ausreichend hohes, positives Zetapotential einstellt. In der Praxis hat es sich gezeigt₁ daß bei pH-Werten im Bereich von 4-5,5 meist ausreichend hohe, positive Zetapotentiale erzielt werden, um Emulsionen mit ausgezeichneter Stabilität zu erhalten, vor allem dann, wenn ein Wirkstoff gewählt wird, der einen pKa-Wert von mindestens 8 besitzt.

Besonders bevorzugt ist für stark hydrophobe Wirkstoffe mit einem pKa-Wert von 8-10 bei einem Wirkstoffgehalt von 0,5-3 Gew.-% die Wahl eines pH-Wertes im Bereich von 4-5,5, wobei die Emulsion 8-25 Gew.-% eines pflanzlichen Öls und 1-2 Gew.-% eines Phospholipids als Emulgator enthalten soll.

Eine beträchtliche Herabsetzung des pH-Wertes über die Erreichung des erwünschten Zetapotentials hinaus ist nicht sinnvoll und birgt die Gefahr in sich, die Öllöslichkeit der Base zu weit zu senken. Das kann zu einer geringeren Verträglichkeit der Zubereitung führen. In der Regel sollte nach Erreichen eines Zetapotentials von +60 mV keine weitere Erhöhung der Acidität vorgenommen werden.

Für die Einstellung des gewünschten pH-Wertes können alle jene Puffersysteme Verwendung finden, die in intravenös zu verabreichenden Arzneimitteln zugelassen sind und die mit dem Wirkstoff keine Reaktionen eingehen. Als solche Puffersysteme können z.B. Acetat-Essigsäure-Puffer, Phosphatpuffer und Citratpuffer genannt werden. Als Fettkomponente können alle üblichen Fette, insbesondere Öle dienen, die für die Bereitung von Fettemulsionen, die für die i.v.-Verabreichung vorgesehen sind, gebräuchlich sind. Pflanzliche Öle, wie Sojaöl, Erdnußöl, Distelöl, Olivenöl, Maiskeimöl, Rüböl, Kokosöl, Sesamöl, Sonnenblumenöl, Palmöl und dergleichen, sind bevorzugt. Der Fettgehalt wird mit 5-30 Gew.-%, zweckmäßig mit 8-25 Gew.-% gewählt, vorzugsweise beträgt er 10 bis 20 Gew.-%. Es ist vorteilhaft, mit zunehmendem Wirkstoffgehalt auch den Fettgehalt zu erhöhen.

Als Phospholipide können sowohl die üblichen Eiphosphatide als auch Sojaphosphatide genannt werden, wobei sowohl solche mit einem Gehalt an rund 80 % Phosphatidylcholin und einem gewissen Anteil an sauren Verunreinigungen, der in einer wirkstofffreien Fettemulsion bei pH 7,4 ein negatives Zetapotential von -40 bis -50 mV ergibt, als auch höher gereinigte Produkte, die zu 90 % und mehr aus einem oder mehreren Phosphatidylcholinen bestehen, Anwendung finden können. Auch reine Phosphatidylcholine, die an sich kaum mehr eine negative Ladung tragen, können eingesetzt werden. Mit diesen sind am leichtesten hohe, positive Ladungen der dispersen Phase zu erreichen. Die Natur des gewählten Phospholipids muß, wie schon erwähnt, ebenfalls bei der Wahl des pH-Wertes berücksichtigt werden. Überraschenderweise ist bei den erfindungsgemäß einzustellenden, sauren pH-Werten der Einfluß höherer Mengen an sauren Bestandteilen im Emulgator wesentlich geringer als bei neutralen pH-Werten, so daß auch mit den üblichen Phospholipiden, die bei pH 7,4 ohne Wirkstoffgehalt Emulsionen mit einem negativen Zetapotential von -40 bis -50 mV ergeben, bei pH-Werten unter 6 meist ausreichend hohe, positive Zetapotentiale, auch solche über +30 mV erzielt werden können. Die Menge an Phospholipid kann zweckmäßigerweise mit 1 bis 2 Gew.-% gewählt werden. Mit dem Phospholipid zusammen können, wenn gewünscht, auch weitere übliche Zusatzemulgatoren, insbesondere solche nicht-ionischen Charakters, verwendet werden.

Als basische, hydrophobe Wirkstoffe, die sich zur Herstellung der erfindungsgemäßen, stabilen Emulsionen eignen, sind alle jene zu nennen, die neben einer Basizität eine ausgeprägte Hydrophobie besitzen und öllöslich sind. Sehr günstige Ergebnisse erhält man mit Wirkstoffen aus der Gruppe der 5-(Phenethylamino)-2-phenylvaleronitrile, wobei Levemopamil besonders bevorzugt ist. Dieser Wirkstoff kann auf erfindungsgemäße Weise zu sehr stabilen, hervorragend verträglichen Darreichungsformen auf Basis einer Fettemulsion als Träger verarbeitet werden. Diese bewirken bei der Verabreichung keinerlei unangenehme Nebenwirkungen und sind darin wäßrigen Darreichungsformen dieses Wirkstoffes überlegen. Auch 2-Dodecyl-5-(methyl-3'-methoxyphenethylamino)-2-3'-methoxyphenylvaleronitril (Anipamil) liefert sehr gute Ergebnisse. Ebenfalls sehr gute Ergebnisse erhält man auch mit Wirkstoffen aus der Gruppe der basische Gruppen tragenden, neuroleptisch wirkenden Phenothiazine, wobei 10-(3-Dimethylaminopropyl)-phenothiazin mit dem pKa-Wert 9,4 (Promazin), 10-(2-Dimethylaminopropyl)-phenothiazin, pKa-Wert 9,1 (Promethazin) und 4-{3-[2-(Trifluormethyl)-phenothiazin-10-yl]-propyl}-1-piperazinoethanol, pKa-Wert 8,05 (Fluphenazine) besonders hervorgehoben werden können. Schließlich können auch basische Lokalanaesthetika, wie z.B. Tetracain, auf erfindungsgemäße Weise in Darreichungsformen übergeführt werden.

Zur Herstellung der erfindungsgemäßen Emulsionen werden das Öl, der Wirkstoff und der Emulgator in die wäßrige Phase eingemischt, die bereits durch das Puffersystem auf den gewünschten, bzw. den durch Vorversuch ermittelten pH-Wert, eingestellt ist. Nach Voremulgierung der Mischung erfolgt dann die Endfertigung durch mehrfache Hochdruckhomogenisierung, die bis zur Erreichung einer durchschnittlichen Partikelgröße von unter 500 nm fortgesetzt wird. Dabei kann der Wirkstoff zunächst im Öl gelöst werden, worauf die resultierende Mischung in eine bereits vordispergierte Mischung des Phospholipids mit der wäßrigen, den Puffer enthaltende Phase eingetragen wird.

Es ist aber ebensogut möglich, den Wirkstoff zunächst mit dem Phospholipid zu mischen, diese Mischung der beiden Stoffe in der wäßrigen Phase zu verteilen und anschließend das Öl darunterzumischen. Für die Hochdruckhomogenisierung können dafür übliche Hochdruckhomogenisatoren oder aber auch Microfluidizer verwendet werden.

In vielen Fällen ist die Einstellung des osmotischen Druckes auf physiologische Verhältnisse wünschenswert, vor allem dann, wenn die erfindungsgemäße Emulsion intravenös verabreicht werden soll. Diese Einstellung kann durch Zusatz einer physiologischen, nichtionischen Substanz erfolgen. Bevorzugt ist hierbei Glycerin.

Die Bestimmung des Zetapotentials in den nachfolgenden Beispielen wurde mit einem käuflichen Meßgerät (Zetasizer 3 der Firma Malvern) durchgeführt, bei dem die Microelektrophorese mit einer Photonen-Korrelations-Spektroskopie, basierend auf einem Helium-Neon-Laser, gekoppelt ist. Die Auswertung erfolgte mittels eines angeschlossenen Computers.

Tabelle 1 zeigt anhand des Beispiels von Levemopamil den Verlauf der Zetapotentiale bei verschiedener Verdünnung auf 0,8 bis 0,002 g/l bzw. 0,08 bis 0,0002 Gew.-% Fett, was, ausgehend von einer 20 %igen Fettemulsion, einer Verdünnung von 1 : 250 bis 1 : 10.000 entspricht. Als Ausgangsemulsionen dienten solche mit verschiedenem Fettgehalt und/oder Wirkstoffgehalt, eingestellt auf pH 5.

Die den Kurven zugrundeliegenden Werte sind folgende (Tab. 1):

| Fettgehalt | Wirkstoffgehalt | Zetapotentiale in mV bei g/l Fett | | | | |
|---|---|---|---|---|---|---|
| | | 0,8 | 0,4 | 0,2 | 0,02 | 0,002 |
| 20 % | 2 % | 55,8 | 49,0 | 41,9 | -4,9 | -6,3 |
| 10 % | 2 % | 53,1 | 45,1 | 35,7 | -17,2 | -5,7 |
| 10 % | 1 % | 57,1 | 49,2 | 42,5 | -9,2 | -6,6 |
| 5 % | 2 % | 53,2 | 48,1 | 41,9 | -11,8 | -1,8 |
| 5 % | 1 % | 51,6 | 42,6 | 34,1 | -14,5 | -3,5 |
| 20 % | 0,5 % | 35,1 | 32,0 | 19,0 | -2,1 | -6,3 |

Der flache Kurvenverlauf bei den Werten, die mit 0,8 bis 0,2 g Fett/l erhalten wurden, unterstreicht die Relevanz der bei 0,8 g Fett/l bzw. 0,08 Gew.-% ermittelten Werte für das Zetapotential für die elektrostatischen Verhältnisse in der unverdünnten Emulsion.

Die erfindinngsgemäßen Emulsionen können für alle jene Zwecke eingesetzt werden, für die flüssige Darreichungsformen pharmazeutischer Wirkstoffe angewendet werden. Sie sind insbesondere für die orale, nasale, pulmonale oder vaginale Verabreichung geeignet. Ein besonderer Vorteil ist, daß sie aufgrund ihrer Zusammensetzung auch für die intravenöse Verabreichung nicht nur geeignet, sondern sogar speziell geeignet sind, wobei sie sich durch gute Verträglichkeit auszeichnen.

### Beispiel 1

12 g Ovothin 200 ®, geeignet zur parenteralen Verabreichung, das zu mehr als 90 % aus Phosphatidylcholin besteht und als wirkstofffreie Fettemulsion in Wasser bei pH 7,4 ein Zetapotential von -20 mV ergibt, wurden in 725 ml einer Acetat-Essigsäure-Pufferlösung (5 mmol/l), die auf pH 5 eingestellt war, bei 50-60°C aufgeschlämnt und zur Einstellung des osmotischen Druckes mit 23 g 86 %igen Glycerins gemischt. Die so erhaltene wäßrige Mischung wurde anschließend einmal bei 200 bar vorhomogenisiert. 20 g Levemopamil wurden ebenfalls bei 50-60°C in 200 g Sojaöl gelöst; die so erhaltene Ölphase wurde portionsweise in der wäßrigen Phase grob dispergiert und 1mal bei 200 bar emulgiert. Nach Wiedereinstellung des pH-Wertes durch Zugabe von Essigsäure auf den Wert 5 wurde der gesamte Ansatz 3mal bei 200 bar homogenisiert. Waren dann noch größere Teilchen feststellbar, so wurden diese durch einen Nachemulgierschritt zerkleinert.

Die so erhaltene Emulsion wurde unter Stickstoffatmosphäre auf Raumtemperatur abkühlen gelassen, über ein 5 µ-Filter filtriert und abgefüllt. Nach 15minütiger Sterilisation bei 121°C erhielt man eine Emulsion mit folgenden Kennzahlen: Zetapotential bei 250facher Verdünnung entsprechend einer Fettkonzentration von 0,08 % + 56 mV, durchschnittliche Partikelgröße 255 nm, Wirkstofffgehalt 2 g/100 ml, das sind 60 mmol/l.

In analoger Vorgangsweise wurden Emulsionen mit pH 4 und pH 6 hergestellt.
- Kennzahlen bei pH 4:: Zetapotential +54 mV, Partikelgröße 210 nm, Wirkstoffkonzentration 2 %
- Kennzahlen bei pH 6:: Zetapotential +37 mV, Partikelgröße 300 nm, Wirkstoffkonzentration 2 %

### Beispiel 2

20 g Anipamil in Form der freien Base wurden mit 200 g Sojaöl gemischt. 12 g des Ovothin ® 200 wurden, wie in Beispiel 1 beschrieben, mit 725 ml einer auf pH 5 eingestellten Na-Acetat-Essigsäure-Pufferlösung dispergiert, mit dem Öl-Wirkstoff-Gemisch versetzt und der Hochdruckhomogenisierung unterworfen.

Nach 15minütigem Sterilisieren im Rotationsautoklaven bei 121°C wurde eine Emulsion mit folgenden Werten erhalten:
Zetapotential +54 mV, durchschnittliche Partikelgröße 300 nm, Wirkstoffgehalt 2 g/100 ml, das sind 60 mmol/l. Anipamilhaltige Emulsionen mit 2 % Wirkstoff in 20 % Fett mit pH-Werten von 4 und 6 wurden in völlig entsprechender Arbeitsweise erhalten.

Hierin ergaben sich folgende Kenngrößen:
- pH 4:: durchschnittliche Partikelgröße 270 nm, Zetapotential +56 mV, Wirkstoffgehalt 2 g/100 ml
- pH 6:: durchschnittliche Partikelgröße 330 nm, Zetapotential +41 mV, Wirkstoffgehalt 2 g/100 ml

### Beispiel 3

12 g Ovothin ® 200 wurden bei 50-60°C in 825 ml einer wäßrigen, auf pH 5 eingestellten Na-Acetat-Essigsäure-Pufferlösung (5 mmol/l) suspendiert, anschließend wurden 20 g Glycerin und dann 20 g Levemopamil-Base eingerührt, worauf der pH-Wert durch Zugabe von Essigsäure auf pH 5 nachkorrigiert wurde. Nach Vorhomogenisieren wurden 100 g Sojaöl portionsweise eingetragen und grob verteilt, worauf man die Mischung 3mal bei 200 bar Druck der Hochdruckhomogenisierung unterwarf. Eventuell noch vorhandene größere Teilchen wurden durch eine anschließende Nachhomogenisierung in kleinere Partikel übergeführt. Nach Abkühlen wurde die Emulsion bei 121°C autoklaviert. Sie zeigte dann folgende Kennzahlen:
Zetapotential +53 mV, die durchschnittliche Partikelgröße betrug 230 nm, der Wirkstoffgehalt 2 g/100 ml, das sind 60 mmol/l, der Fettgehalt betrug 10 Gew.-%.

### Beispiel 4

12 g eines 90 % Phosphatidylcholin enthaltenden Eilecithins, das bei pH 7,4 ein Zetapotential von -20 mV ergab (Ovothin ® 200) und 20 g Glycerin wurden in 875 ml einer auf pH 5 eingestellten Na-Acetat-Essigsäure-Pufferlösung, wie in Beispiel 1 beschrieben, dispergiert und bei 50-60°C portionsweise mit einer Mischung aus 20 g Levemopamil-Base und 50 g Sojaöl versetzt. Das Gemisch wurde zunächst bei 200 bar vorhomogenisiert und nach Feinkorrektur des pH-Wertes bei einem Druck von 140 bar der Homogenisierung bis zu einer durchschnittlichen Partikelgröße von 220 nm unterworfen. Nach der gemäß Beispiel 1 durchgeführten Abfüllung und Sterilisation ergab die Emulsion ein Zetapotential von +53 mV. Die durchschnittliche Partikelgröße lag bei 220 nm. Der Wirkstoffgehalt betrug 2 g/100 ml, das sind 60 mmol/l, der Fettgehalt betrug 5 Gew.-%.

### Beispiel 5

1 g Levemopamil-Base wurde mit 5 g Sojaöl gemischt. Die Mischung wurde in eine vordispergierte Mischung aus 1,2 g eines hochgereinigten Lecithins, 2 g Glycerin und 88 ml einer auf pH 5 eingestellten Pufferlösung gegeben und mit ansteigendem Druck zwischen 140 und 200 bar einer Hochdruckhomogenisierung unterzogen. Nach 15minütigem Sterilisieren im Rotationsautoklaven bei 121°C wurde eine Emulsion mit folgenden Werten erhalten:
Zetapotential +52 mV, durchschnittliche Partikelgröße 230 nm, Wirkstoffgehalt 1 g/100 ml entsprechend 30 mmol/l, der Fettgehalt betrug 5 Gew.-%.

### Beispiel 6

12 g Eilecithin mit einem Zetapotential von -20 mV wurden in 730 ml einer mit Glycerin isotonisierten Essigsäure-Acetat-Pufferlösung (5 mmol/l) von pH 5 vordispergiert (Ultraturrax); in diese Vordispersion wurden 20 g Promethazinbase eingearbeitet, anschließend wurde die daraus resultierende Mischung mit 200 g Sojaöl versetzt und in einem Hochdruckhomogenisator 4mal bei 200 bar emulgiert. Die Emulsion wies nach 15minütigem Autoklavie-ren unter Rotation bei 121°C folgende Kenngrößen auf:
Zetapotential +46 mv, durchschnittliche Partikelgröße 250 nm, Wirkstoffgehalt 20 g/l, der Fettgehalt betrug 20 Gew.-%.

In entsprechender Weise wurden promethazinhaltige Emulsionen mit pH 4 und pH 6 angefertigt.
- Kenngrößen bei pH 4:: Zetapotential +52 mV, durchschnittliche Partikelgröße 250 nm, Wirkstoffgehalt 20 g/l
- Kenngrößen bei pH 6:: Zetapotential +41 mV, durchschnittliche Partikelgröße 280 nm, Wirkstoffgehalt 20 g/l

### Beispiel 7

Wie in Beispiel 6 beschrieben, wurden 2 g Promazin-Base in 73 ml Puffer-Glycerinlösung mit pH 5, die 1,2 g des gleichen Eilecithins, wie in Beispiel 6 verwendet, enthielt, mit 20 g Sojaöl vordispergiert und anschließend bei 140 bis 200 bar unter jeweils ansteigendem Druck 4mal einer Hochdruckhomogenisierung unterworfen. Nach Autoklavieren ergab sich eine stabile Emulsion mit den Kennzahlen: Zetapotential +44 mV, durchschnittliche Partikelgröße 230 nm, Wirkstoffgehalt 2 %, der Fettgehalt betrug 20 Gew.-%. Die entsprechend hergestellten Emulsionen mit pH 4 bzw. pH 6 wiesen bei sonst gleichen Kennwerten Zetapotentiale von +56 mV (pH 4) und +39 mV (pH 6) auf.

### Beispiel 8

Zur Emulsionsbereitung wurden 10 g Levemopamil in 100 g Sojaöl gelöst. Diese Mischung wurde unter einem Schnellrührer bei etwa 60°C in 825 ml einer Acetat-Pufferlösung, die auf pH 5 eingestellt und mit Glycerin isotonisiert war, sowie 12 g Ovothin ® 200 enthielt, eindispergiert und in 4 Schritten bei 200 bar der Hochdruckhomogenisierung unterworfen.

Nach Abfüllen und Sterilisieren im Rotationsautoklaven bei 121°C über 15 Min. wurden an der Emulsion folgende Kenndaten ermittelt:
Zetapotential +57 mV, durchschnittliche Partikelgröße 300 nm, Wirkstoffgehalt 10 g/l entsprechend 30 mmol/l, der Fettgehalt betrug 10 Gew.-%.

### Beispiel 9

Es wurden 20 g Levemopamil, in 200 g Sojaöl aufgelöst, unter einem Schnellrührer in 725 ml einer mit Glycerin isotonisierten, auf pH 5 eingestellten Acetat-Pufferlösung, die 12 g eines Eilecithins mit einem Gehalt an Phosphatidylcholin von 80 % und einem Zetapotential bei pH 7,4 von -40 bis -50 mV (Lipoid E 80 ®) enthielt, eingearbeitet und anschließend in 4 Schritten bei 160-180 bar der Hochdruckhomogenisierung unterzogen.

Die derart hergestellte Emulsion wurde über 5 µ-Filter filtriert, abgefüllt und bei 121°C 15 Min. lang unter Rotation sterilisiert. An der fertigen Emulsion wurden folgende Werte gemessen:
Zetapotential +44 mV, durchschnittliche Teilchengröße 300 nm, Wirkstoffgehalt 20 g/l, der Fettgehalt betrug 20 Gew.-%.

## Patentansprüche (Patentansprüche für folgende(n) Vertragsstaat(en): AT, BE, CH, DE, DK, FR, GB, IT, LI, NL)

1. Stabile, zur pharmazeutischen, insbesondere intravenösen Verabreichung geeignete Emulsion vom Typ Öl-in-Wasser, mit einem Fettgehalt von 5-30 Gew.-% und einem Gehalt an 0,5-2 Gew.-% eines Phospholipids als Emulgator, die in der Lipidphase einen eine oder mehrere basische Gruppen tragenden, hydrophoben, pharmazeutischen Wirkstoff in fein verteilter und/oder gelöster Form enthält, dadurch gekennzeichnet, daß der Wirkstoff öllöslich ist und einen pKa-Wert von mindestens 7,5 aufweist, die wäßrige Phase durch Gehalt eines physiologisch verträglichen Puffersystems auf einen pH-Wert im sauren Bereich eingestellt ist und die disperse Phase nach Verdünnung der verabreichungsfertigen Emulsion auf einen Fettgehalt von 0,08 Gew.-% ein positives Zetapotential von mindestens +15 mV ergibt.

2. Emulsion gemäß Anspruch 1, dadurch gekennzeichnet, daß die disperse Phase nach Verdünnung der verabreichungsfertigen Emulsion auf einen Fettgehalt von 0,08 Gew.-% ein positives Zetapotential von mindestens +30 mV ergibt.

3. Emulsion gemäß den Ansprüchen 1 oder 2, dadurch gekennzeichnet, daß der Wirkstoff einen pKa-Wert von mindestens 8 besitzt.

4. Emulsion gemäß den Ansprüchen 2 und 3, dadurch gekennzeichnet, daß sie 8-25 Gew.-% eines pflanzlichen Öls, 1-2 Gew.-% des Phospholipids und 0,5-3 Gew.-% eines stark hydrophoben Wirkstoffes mit einem pKa-Wert von 8-10 enthält und der pH-Wert der wäßrigen Phase durch das Puffersystem auf 4-5,5 eingestellt ist.

5. Emulsion gemäß den Ansprüchen 1 bis 4, dadurch gekennzeichnet, daß der Wirkstoff aus der Gruppe der 5-(Phenethylamino)-2-phenyl-valeronitrile ausgewählt ist.

6. Emulsion gemäß Anspruch 5, dadurch gekennzeichnet, daß der Wirkstoff (-)-(S)-2-Isopropyl-5-(methylphenethylamino)-2-phenyl-valeronitril ist.

7. Emulsion gemäß Anspruch 5, dadurch gekennzeichnet, daß der Wirkstoff 2-Dodecyl-5-(methyl-3'-methoxyphenethylamino)-2-3'-methoxyphenyl-valeronitril ist.

8. Emulsion gemäß den Ansprüchen 1 bis 4, dadurch gekennzeichnet, daß der Wirkstoff aus der Gruppe der basische Gruppen tragenden, neuroleptisch wirkenden Phenothiazine ausgewählt ist.

9. Verfahren zur Herstellung von Emulsionen gemäß den Ansprüchen 1 bis 8, dadurch gekennzeichnet, daß die Fettkomponente mit einem das Phospholipid enthaltenden, wäßrigen Medium, das durch Zusatz eines physiologisch verträglichen Puffersystems auf einen sauren pH-Wert eingestellt ist, der gemäß Vorversuch nach Verdünnung der fertigen Mischung auf einen Fettgehalt von 0,08 Gew.-% ein Zetapotential von mindestens +15 mV ergibt und dessen osmotischer Druck gewünschtenfalls durch Zugabe einer physiologischen, nichtionischen Substanz auf physiologische Werte gebracht ist, gemischt wird, wobei der basische Gruppen tragende, hydrophobe, öllösliche Wirkstoff gelöst in der Ölkomponente oder in Mischung mit dem Phospholipid in das System eingebracht wird, worauf die resultierende Mischung in einem oder mehreren Schritten bis zum Erreichen einer durchschnittlichen Partikelgröße von unter 500 nm unter hohem Druck homogenisiert wird.

10. Emulsion gemäß den Ansprüchen 1 bis 8 zur Anwendung als Arzneimittel, insbesondere zur intravenösen Verabreichung.

## Patentansprüche (Patentansprüche für folgende(n) Vertragsstaat(en): ES)

1. Verfahren zur Herstellung einer stabilen, zur pharmazeutischen, insbesondere intravenösen Verabreichung geeigneten Emulsion vom Typ Öl-in-Wasser, mit einem Fettgehalt von 5-30 Gew.-% und einem Gehalt an 0,5-2 Gew.-% eines Phospholipids als Emulgator, die in der Lipidphase einen eine oder mehrere basische Gruppen tragenden, hydrophoben, pharmazeutischen Wirkstoff in fein verteilter und/oder gelöster Form enthält, dadurch gekennzeichnet, daß die Fettkomponente mit einem, das Phospholipid enthaltenden wäßrigen Medium, das durch Zusatz eines physiologisch verträglichen Puffersystems auf einen sauren pH-Wert eingestellt ist, der gemäß Vorversuch nach Verdünnen der fertigen Mischung auf einen Fettgehalt von 0,08 Gew.-% ein positives Zetapotential von mindestens +15 mV ergibt, und dessen osmotischer Druck gewünschtenfalls durch Zugabe einer physiologischen, nicht-ionischen Substanz auf physiologische Werte gebracht ist, gemischt wird, wobei der Wirkstoff, der öllöslich ist und einen pKa-Wert von mindestens 7,5 aufweist, gelöst in der Ölkomponente oder in Mischung mit dem Phospholipid in das System eingebracht wird, worauf die resultierende Mischung in einem oder mehreren Schritten bis zum Erreichen einer durchschnittlichen Partikelgröße von unter 500 nm unter hohem Druck homogenisiert wird und dabei ein geringer Teil des Wirkstoffes in die wäßrige Phase übergeführt und dort unter dem Einfluß des sauren pH-Wertes zumindest zum Teil ionisiert wird.

2. Verfahren gemäß Anspruch 1, dadurch gekennzeichnet, daß die disperse Phase nach Verdünnung der verabreichungsfertigen Emulsion auf einen Fettgehalt von 0,08 Gew.-% ein positives Zetapotential von mindestens +30 mV ergibt.

3. Verfahren gemäß den Ansprüchen 1 und 2, dadurch gekennzeichnet, daß der Wirkstoff einen pKa-Wert von mindestens 8 besitzt.

4. Verfahren gemäß den Ansprüchen 2 und 3, dadurch gekennzeichnet, daß zur Herstellung der Emulsion 8-25 Gew.-% eines pflanzlichen Öls, 1-2 Gew.-% des Phospholipids und 0,5-3 Gew.-% eines stark hydrophoben Wirkstoffes mit einem pKa-Wert von 8-10, Prozentangaben jeweils bezogen auf die fertige Emulsion, eingesetzt werden, und die wäßrige Phase durch das Puffersystem auf einen pH-Wert von 4-5,5 eingestellt wird.

5. Verfahren gemäß den Ansprüchen 1 bis 4, dadurch gekennzeichnet, daß ein Wirkstoff aus der Gruppe der 5-(Phenethylamino)-2-phenyl**-**valeronitrile eingesetzt wird.

6. Verfahren gemäß Anspruch 5, dadurch gekennzeichnet, daß als Wirkstoff (-)-(S)-2-Isopropyl-5-(methylphenethylamino)-2-phenyl-valeronitril eingesetzt wird.

7. Verfahren gemäß Anspruch 5, dadurch gekennzeichnet, daß als Wirkstoff 2-Dodecyl-5-(methyl-3'-methoxyphenethylamino)-2-3'-methoxyphenylvaleronitril eingesetzt wird.

8. Verfahren gemäß den Ansprüchen 1 bis 4, dadurch gekennzeichnet, daß als Wirkstoff ein solcher aus der Gruppe der basische Gruppen tragenden, neuroleptisch wirkenden Phenothiazine eingesetzt wird.

## Claims (Claims for the following Contracting State(s): AT, BE, CH, DE, DK, FR, GB, IT, LI, NL)

1. A stable oil-in-water emulsion which is suitable for pharmaceutical, in particular intravenous, administration, which has a fat content of 5-30 % by weight and a content of 0.5-2 % by weight of a phospholipid as emulsifier, and which contains, finely dispersed and/or dissolved in the lipid phase, a hydrophobic pharmaceutical active substance which has one or more basic groups, wherein the active substance is oil-soluble and has a pKa of not less than 7.5, the aqueous phase is adjusted to a pH in the acid range by the presence of a physiologically tolerated buffer system, and the disperse phase gives, after dilution of the emulsion ready for administration to a fat content of 0.08 % by weight, a positive zeta potential of not less than +15 mV.

2. An emulsion as claimed in claim 1, wherein the disperse phase gives, after dilution of the emulsion ready for administration to a fat content of 0.08 % by weight, a positive zeta potential of not less than +30 mV.

3. An emulsion as claimed in claim 1 or 2, wherein the active substance has a pKa of not less than 8.

4. An emulsion as claimed in claims 2 and 3, which contains 8-25 % by weight of a vegetable oil, 1-2 % by weight of the phospholipid and 0.5-3 % by weight of a strongly hydrophobic active substance with a pKa of 8-10, and wherein the pH of the aqueous phase is adjusted to 4-5.5 by the buffer system.

5. An emulsion as claimed in claims 1 to 4, wherein the active substance is selected from the group of 5- (phenethylamino)-2-phenylvaleronitriles.

6. An emulsion as claimed in claim 5, wherein the active substance is (-)-(S)-2-isopropyl-5-(methylphenethylamino)-2-phenylvaleronitrile.

7. An emulsion as claimed in claim 5, wherein the active substance is 2-dodecyl-5-(methyl-3'-methoxyphenethylamino)-2-3'-methoxyphenylvaleronitrile.

8. An emulsion as claimed in claims 1 to 4, wherein the active substance is selected from the group of phenothiazines which have basic groups and neuroleptic activity.

9. A process for the preparation of emulsions as claimed in claims 1 to 8, which comprises mixing the fat component with an aqueous medium which contains the phospholipid and is adjusted by addition of a physiologically tolerated buffer system to an acidic pH which, according to a preliminary test, gives, after dilution of the finished mixture to a fat content of 0.08 % by weight, a zeta potential of not less than +15 mV and whose osmotic pressure is brought to physiological values if desired by adding a physiological, non-ionic substance, where the hydrophobic, oil-soluble active substance which has basic groups is introduced into the system dissolved in the oil component or mixed with the phospholipid, after which the resulting mixture is homogenized under high pressure in one or more steps until an average particle size of less than 500 nm is reached.

10. An emulsion as claimed in claims 1 to 8 for use as drug, in particular for intravenous administration.

## Claims (Claims for the following Contracting State(s): ES)

1. A process for the preparation of a stable oil-in-water emulsion which is suitable for pharmaceutical, in particular intravenous, administration, which has a fat content of 5-30 % by weight and a content of 0.5-2 % by weight of a phospholipid as emulsifier, and which contains, finely dispersed and/or dissolved in the lipid phase, a hydrophobic pharmaceutical active substance which has one or more basic groups, which comprises mixing the fat component with an aqueous medium which contains the phospholipid and is adjusted by addition of a physiologically tolerated buffer system to an acidic pH which, according to a preliminary test, gives, after dilution of the finished mixture to a fat content of 0.08 % by weight, a positive zeta potential of not less than +15 mV and whose osmotic pressure is brought to physiological values if desired by adding a physiological, non-ionic substance, where the oil-soluble active substance which has a pKa of not less than 7.5 is introduced into the system dissolved in the oil component or mixed with the phospholipid, after which the resulting mixture is homogenized under high pressure in one or more steps until an average particle size of less than 500 nm is reached, and moreover a small portion of the active substance is transferred into the aqueous phase and then at least partly ionized under the influence of the acidic pH.

2. A process as claimed in claim 1, wherein the disperse phase gives, after dilution of the emulsion ready for administration to a fat content of 0.08 % by weight, a positive zeta potential of not less than +30 mV.

3. A process as claimed in claims 1 and 2, wherein the active substance has a pKa of not less than 8.

4. A process as claimed in claims 2 and 3, wherein 8-25 % by weight of a vegetable oil, 1-2 % by weight of the phospholipid and 0.5-3 % by weight of a strongly hydrophobic active substance with a pKa of 8-10, percentage data in each case based on the finished emulsion, are employed to prepare the emulsion, and the aqueous phase is adjusted to a pH of 4-5.5 by the buffer system.

5. A process as claimed in claims 1 to 4, wherein an active substance from the group of 5-(phenethylamino)-2-phenylvaleronitriles is employed.

6. A process as claimed in claim 5, wherein (-)-(S)-2-isopropyl-5-(methylphenethylamino)-2-phenylvaleronitrile is employed as active substance.

7. A process as claimed in claim 5, wherein 2-dodecyl-5-(methyl)-3'-methoxyphenethylamino)-2-3'-methoxyphenylvaleronitrile is employed as active substance.

8. A process as claimed in claims 1 to 4, wherein the active substance employed is one from the group of phenothiazines which have basic groups and neuroleptic activity.

## Revendications (Revendications pour l'(les) Etat(s) contractant(s) suivant(s): AT, BE, CH, DE, DK, FR, GB, IT, LI, NL)

1. Emulsion stable, appropriée pour l'administration de produits pharmaceutiques, en particulier par voie intraveineuse, de type huile-dans-l'eau, ayant une teneur en matière grasse de 5-30 % en poids et une teneur en un phospholipide en tait qu'émulsifiant de 0,5-2 % en poids, qui contient, dans la phase lipidique, une substance active pharmaceutique hydrophobe, portant un ou plusieurs groupes basiques, sous une forme finement divisée et/ou dissoute, caractérisée en ce que la substance active est oléosoluble et présente un pKa d'au moins 7,5, la phase aqueuse est ajustée à un pH acide à l'aide d'un système tampon physiologiquement acceptable, et la phase dispersée, après dilution de l'émulsion prête à l'administration à une teneur en matière grasse de 0,08 % en poids, presente un potentiel zéta positif d'au moins +15 mV.

2. Emulsion selon la revendication 1, caractérisée en ce que la phase dispersée, après dilution de l'émulsion prête à l'administration à une teneur en matière grasse de 0,08 % en poids, présente un potentiel zéta positif d'au moins +30 mV.

3. Emulsion selon la revendication 1 ou 2, caractérisée en ce que la substance active présente un pKa d'au moins 8.

4. Emulsion selon les revendications 2 et 3, caractérisée en ce qu'elle contient 8-25 % en poids d'une huile végétale, 1-2 % en poids du phospholipide et 0,5-3 % en poids d'une substance active fortement hydrophobe ayant un pKa de 8-10 et que le pH de la phase aqueuse est ajusté à 4,5-5 à l'aide du système tampon.

5. Emulsion selon les revendications 1 à 4, caractérisée en ce que la substance active est choisie dans le groupe des 5-(phénéthylamino)-2-phénylvaléronitriles.

6. Emulsion selon la revendication 5, caractérisée en ce que la substance active est le (-)-(S)-2-isopropyl-5-(méthylphénéthylamino)-2-phénylvaléronitrile.

7. Emulsion selon la revendication 5, caractérisée en ce que la substance active est le 2-dodécyl-5-(méthyl-3'-méthoxyphénéthylamino)-2-3'-méthoxyphényl-valéronitrile.

8. Emulsion salon les revendications 1 à 4, caractérisée en ce que la substance active est choisie dans le groupe des phénothiazines ayant une action nauroleptique, portant des groupes basiques.

9. Procédé pour la préparation d'émulsions selon les revendications 1 à 8, caractérisé en ce que le composant gras est mélangé avec un milieu aqueux contenant le phospholipide, dont le pH est ajusté à une valeur acide par addition d'un système tampon physiologiquement acceptable, qui présente, selon un essai préalable après dilution du mélange fini à une teneur en matière grasse de 0,08 % en poids, un potentiel zéta d'au moins +15 mV, et dont la pression osmotique est ajustée à une valeur physiologique, éventuellement par addition d'une substance physiologique non ionique, la substance active oléosoluble, hydrophobe, portant des groupes basiques, étant introduite dans le système soit en solution dans le composant huile, soit en mélange avec le phospholipide. après quoi le mélange obtenu est homogénéisé sous pression élevée, en une ou plusieurs étapes, jusqu'à obtention d'une taille moyenne de particule Inférieure à 500 nm.

10. Emulsion selon les revendications 1 à 8, destinée à l'utilisation comme médicament, en particulier pour administration par voie intraveineuse.

## Revendications (Revendications pour l'(les) Etat(s) contractant(s) suivant(s): ES)

1. Procédé de préparation d'une émulsion stable, appropriée pour l'administration de produits pharmaceutiques, en particulier par voie Intraveineuse, de type huile-dans-l'eau, ayant une teneur en matière grasse de 5-30 % en poids et une teneur en un phospholipide en tant qu'émulsifiant de 0,5-2 % en poids, qui contient, dans la phase lipidique, une substance active pharmaceutique hydrophobe, portant un, ou plusieurs groupes basiques, sous une forme finement divisée et/ou dissoute, caractérisé en ce que le composent gras est mélangé avec un milieu aqueux contenant le phospholipide. dont le pH est ajusté à une valeur acide par addition d'un système tampon physiologiquement acceptable, qui présente, selon un essai préalable après dilution du mélange fini à une teneur an matière grasse de 0,08 % en poids, un potentiel zéta d'au moins +15 mV, et dont la pression osmotique est ajustée à une valeur physiologique, éventuallement par addition d'une substance physiologique non ionique, la substance active oléosoluble, hydrophobe, portant des groupes basiques, qui est oléosoluble et présente un pKa d'au moins 7,5, étant introduite dans le système soit en solution dans le composant huile, soit en mélange avec le phospholipide, après quoi le mélange obtenu est homogénéisé sous pression élevée, en une ou plusieurs étapes, jusqu'à obtention d'une taille moyenne de particule inférieure à 500 nm, une faible partie de la substance active étant transférée ainsi dans la phase aqueuse et là, sous l'action du pH acide, ionisée au moins en partie.

2. Procédé selon la revendication 1, caractérisé en ce que la phase dispersée, après dilution de l'émulsion prête à l'administration à une teneur en matière grasse de 0,08 % en poids, présente un potentiel zéta positif d'au moins +30 mV.

3. Procédé selon la revendication 1 ou 2, caractérisé en ce que la substance active présente un pKa d'au moins 8.

4. Procédé selon les revendications 2 et 3, caractérisé en ce que pour la préparation de l'émulsion, on utilise 8-25 % en poids d'une huile végétale, 1 - 2 % en poids du phospholipide et 0,5-3 % en poids d'une substance active fortement hydrophobe ayant un pKa de 8-10, les proportions indiquées étant toujours calculées par rapport à l'émulsion finie, et que le pH de la phase aqueuse est ajusté à 4,5-5 à l'aide du système tampon.

5. Procédé selon les revendications 1 à 4, caractérisé en ce que l'on utilise une substance active choisie dans le groupe des 5-(phénéthylamino)-2-phényl-valéronitriles.

6. Procédé selon la revendication 5, caractérisé en ce comme substance active, on utilise le (-)-(S)-2-isopropyl-5-(méthylphénéthylamino)-2-phényl-valéronitrile.

7. Procédé selon la revendication 5, caractérisé en ce que comme substance active, on utilise le 2-dodécyl-5-(méthyl-3'-méthoxyphénéthylamino)-2-3'-méthoxyphényl-valéronitrile.

8. Procédé selon les revendications 1 à 4, caractérisé en ce que comme substance active, on utilise une substance choisie dans le groupe des phénothiazines ayant une action neuroleptique, portant des groupes basiques.
